# EUROPEAN PATENT APPLICATION

(11) **EP 2 644 093 A1**
(43) Date of publication of application: **02.10.2013**
(21) Application number: 13161952.0
(22) Date of filing: 02.04.2013
(51) Int. Cl.: A61B 5/055, A61B 5/20

(54) **Method for diagnosis of acute kidney injury using magnetic resonance imaging**

(30) Priority: 30.03.2012 EP 12162336; 12.06.2012 EP 12171687
(71) Applicant: Max-Delbrück-Centrum für Molekulare Medizin, 13125 Berlin (DE)
(72) Inventor: Pohlmann, Andreas, 16321 Bernau (DE); Lajos, Markó, 13156 Berlin (DE); Niendorf, Thoralf, 52074 Aachen (DE); Müller, Domenik N., 10405 Berlin (DE)
(74) Representative: Lange, Sven

(57) **Abstract**

The invention relates to a non-invasive method for the diagnosis of acute kidney injury (AKI) comprising T2- and/or T2*-weighted magnetic resonance imaging (MRI) analysis of one or more kidneys of a subject.

## Description

The invention relates to the field of kidney disorders and methods for detecting acute kidney injury. The invention relates to a non-invasive method for the diagnosis of acute kidney injury (AKI) comprising T2- and/or T2*-weighted magnetic resonance imaging (MRI) analysis of one or more kidneys of a subject.

### BACKGROUND OF THE INVENTION

Clinically acute kidney injury (AKI) is generally defined as a sudden impairment of kidney function, often as a rapid reduction in the glomerular filtration rate (GFR) [references a1, a2]. AKI is not only the most common kidney disease, but is also associated with an increased morbidity and mortality of patients in normal care (+10%) and intensive care (+30 to +50%) [a3-a6]. Furthermore, the number of AKI cases in hospitals has increased by a factor of 20 in the last 25 years in the United States alone [a7].

Acute kidney injury (AKI) can be caused by kidney hypoperfusion or temporary interruption of blood flow in various clinical settings [1-3]. This ischemia/reperfusion injury (I/RI) is characterized by mismatch of local tissue oxygen supply and local cellular energy demand and results in sublethal or lethal injuries to the tubular epithelium and neighbouring cell structures which is clinically detected as compromised glomerular filtration rate [4,5].

AKI is also a common origin of chronic kidney disease (CKD), which can lead to renal failure and the need for renal replacement therapy (hemodialysis, hemofiltration and kidney transplantation)[a1, a9]. Even in the final stage, i.e. kidney transplantation, the inevitable ischemia during this procedure may result in AKI of the transplant.

Patients with AKI have a poor prognosis, so that an early and thorough diagnosis that allows taking preventive measures is of key importance [a8]. Serum creatinine and diuresis (urine volume in ml/kg body weight /h) are the established markers for AKI [a1], which are currently used to diagnose and categorize AKI into different stages. The RIFLE criteria and AKIN criteria, which are based on the combination of these two markers, are closely correlated with severity of AKI and mortality [a4, a10-a12]. In an attempt to correct for age, sex and body weight - all parameters that influence serum creatinine levels [a1] - the serum creatinine is translated into the estimated glomerular filtration rate (eGFR) [a2, a13]. The RIFLE classification uses changes in serum creatinine and eGFR over one week, with the least severe degree of AKI starting at +50% creatinine or -25% eGFR. The further developed AKIN classification defines and increase in creatinine ≥ 26.4 µmol/l or 50% within 48 hours as the lowest severity level and hence allows for a more rapid diagnosis [a2]. Yet in clinical practice, both classification methods do not differ substantially in their diagnostic value [a4, a14].

Recently, new molecular biomarkers have been developed and have been (or still are being) tested clinically. Particularly promising among those new biomarkers are Cystatin C and NGAL (Neutrophil Gelatinase-associated Lipocalin) in serum or urine [a15, a16]. Cystatin C, NGAL, and KIM-1 (Kidney Injury Molecule 1) are earlier and more sensitive markers for AKI than serum creatinine [a1].

Early and thorough diagnosis of AKI is essential [a8], but the crux of the matter is that "the two established markers to prove evidence of AKI, the serum creatinine level and the diuresis, are not suitable for the early diagnosis of AKI" [a2].

Reasons for this include the low sensitivity of creatinine levels: only from a functional impairment of around 50% (equivalent to one kidney failing entirely) creatinine levels increase significantly. Moreover, the age, sex, and muscle mass affect creatinine levels considerably, just as some medications do [a15]. The calculation of the eGFR can be performed by several formulas; one of the most widely accepted is the Modification of Diet in Renal Disease (MDRD) method [a13], which includes the age, sex and body weight of the patient, but uses a standardized formula, which inevitably results in a crude estimate. A particular weakness of the RIFLE and AKIN criteria is the interpretation of results in cases where the creatinine (or diurese) is already markedly increased (or decreased respectively) at the first evaluation [a2].

The new biomarker NGAL has a high prognostic value, but the fact that it is not specific to the kidneys, but can also be found for instance in the lung, stomach, and intestine is potentially confounding [a1]. This problem could be circumvented by measuring NGAL in biopsies taken from the kidney - however, invasive biopsies for this purpose (with the associated risk of kidney infection) would be inadequate in clinical routine. Cystatin C has the same drawback, as it is also not specific to the kidneys (present in all cells), and normal levels are age-dependent.

MRI has many advantages compared to systemic markers, obtained from serum or urine, and allows for the direct assessment of the kidneys and substructures within them. While changes in MRI images under pathophysiological conditions have been observed in preclinical and clinical studies (e.g. oxygenation dependent contrast changes [a17, a19, 25] the correlation between these changes seen with MRI and the severity of the AKI remains unknown. This limits the diagnostic value of MRI for AKI.

As mentioned above, several methods are available to diagnose and classify acute kidney injuries. These methods include the established serum creatinine, eGFR and diuresis as well as the novel molecular biomarkers NGAL, KIM-1 and Cystatin C. Further potential biomarkers are being developed and are still in early experimental stages.

Although the described methods are able to detect an AKI pathogenesis at a certain stage, their main drawback is the low sensitivity (creatinine), potential bias by elevated levels at the first evaluation, and the potentially confounding co-factors. Novel biomarkers like NGAL suffer from non-specificity, and others require too much time (like the classical creatinine assay) to be suitable for early diagnosis.

Despite substantial progress in the field of AKI including biomarker discovery and renal replacement therapy dosing, there is an unmet need to provide improved means for detection and characterisation during the initial phase of ischemic AKI. Treatments targeting processes operative before the onset of sublethal or lethal cellular injuries would have great clinical impact. Animal models are well suited to study mechanisms involved in the pathogenesis of renal ischemia/reperfusion injury and helped to define the injury phases of initiation, extension and regeneration [6-8]. The imbalance between oxygen demand and supply is considered to be the initiating step in the pathophysiologic cascade of events [9-11]. Renal tissue oxygen tension (pO2) as determined by invasive in-vivo measurements in the first hour after reperfusion may be decisive for renal damage at 48 hours [12,13]. However, lack of adequate diagnostic tools capable to determine spatio-temporal distribution of oxygenation during the phases of renal ischemia/reperfusion precluded elucidation of events taking place immediately after initiation of ischemia and reperfusion.

Non-invasive in vivo imaging provides the ability to elucidate spatio-temporal pathophysiological changes in the kidney by monitoring the early phases of ischemia and reperfusion. Magnetic resonance imaging (MRI) meets the needs of non-invasive renal in vivo imaging. MRI, also referred to as nuclear magnetic resonance (NMR) imaging, is a non-invasive imaging technique, encompassing the detection of atomic nuclei with magnetic fields and radio frequency (RF) radiation. MRI does not employ x-rays or radioactive tracers and is therefore suitable for repeated measurements and longitudinal studies.

Parametric MRI can provide maps of the MR relaxation parameters T2* and T2, which are known to be blood oxygenation level-dependent (BOLD) [13]. However, MRI is not being used in the routine diagnosis of AKI. None of the earlier work in MRI analysis of the kidney has applied high temporal resolution continuous parametric MRI based in-vivo monitoring during ischemia - and in particular during early reperfusion of the kidney - in order to assess T2-weighted MRI for the early diagnosis of AKI.

Earlier work has disclosed MRI analysis of renal oxygenation levels (Li et al., Magn Reson Imaging Clin N Am, 2008 16(4):613). Li et al however make no mention of early AKI detection. Dendrimer-enhanced MRI has been applied as a diagnostic tool of sepsis-induced acute renal failure (Dear et al., Kidney International, 67 (20005), 2159-2167). Dear et al utilise T1-weighted MRI and focus only on anatomical changes of the kidney 6 or 20 hours after antibiotic treated cecal ligation and puncture sepsis model via use of contrast agent. No mention is made of oxygenation-dependent MR measurement.

### SUMMARY OF THE INVENTION

In light of the prior art the technical problem underlying the invention was the provision of means for the early detection or diagnosis of AKI that do not exhibit the disadvantages of the prior art.

This problem is solved by the features of the independent claims. Preferred embodiments of the present invention are provided by the dependent claims.

Therefore, an object of the invention is to provide a non-invasive method for the diagnosis of acute kidney injury (AKI) comprising T2- and/or T2*-weighted magnetic resonance imaging (MRI) of one or more kidneys of a subject. An object of the invention is also the use of T2-and/or T2*-weighted magnetic resonance imaging (MRI) of one or more kidneys of a subject in a non-invasive method for the diagnosis of acute kidney injury (AKI).

Ischemia/reperfusion (I/R) injury, a consequence of kidney hypoperfusion or temporary interruption of blood flow, is a common cause of acute kidney injury (AKI). The present invention relates in a preferred embodiment to non-invasive in vivo parametric magnetic resonance imaging (MRI) in order to elucidate the spatio-temporal pathophysiological changes in the kidney, in particular changes in kidney tissue oxygenation, by monitoring the MR relaxation parameters T2* and T2, thereby providing a method for the early detection of AKI.

The invention demonstrates fast continuous T2*/T2 mapping throughout renal I/R. MRI is combined in one embodiment of the examples with a remotely controlled I/R model and a segmentation model based semi-automated quantitative analysis. This technique enables the detailed assessment of in vivo changes in all kidney regions during ischemia and early reperfusion.

Significant changes in T2* and T2 are observed shortly after induction of renal ischemia and during the initial reperfusion phase. The present invention shows for the first time that continuous and high temporal resolution parametric MRI is feasible for in-vivo monitoring and characterization of I/R induced AKI.

A preferred embodiment of the invention relates to the use of parametric mapping of T2* and T2 values in the method of the present invention. This approach is also known as parametric magnetic resonance imaging. Parametric mapping relates to generating and/or capturing a series of MR images acquired with different T2 or T2* weighting, which can be adjusted in the MRI method. This approach avoids any bias that may be inherent in the conventional approach to apply a single T2 or T2* weighting (which may be sub-optimal for the analysis), as it allows for the direct calculation of the parameters T2 and T2*. These parametric maps of absolute T2* and T2 values can be calculated using a series of MR images acquired at different echo times. For each image pixel a mono-exponential function is fitted to the signal intensities at different echo times, yielding time constants (T2*/T2) that quantitatively describe these MR properties of the local tissue. The T2*- and T2-weighted images series may be acquired with multi-echo gradient-echo or multi-echo spin-echo MR pulse sequences respectively.

The methods of the prior art are based substantially on the production of single images using MRI and subsequent subjective analysis of the obtained images, in particular a visual analysis of the morphological structure of the kidneys, the state of kidney tissue or any structural damage induced by kidney failure or injury. When using only a single T2- or T2*-weighting, the images obtained are dependent on the employed T2- or T2*-weighting (which might also be either too strong or too weak) and may be biased by various variable technical factors, such as the power adjustments of the MR system or the distance of the kidney to the radio frequency antenna. This could lead to artefacts in analysis and incorrect diagnosis.

The use of parametric mapping according to the present invention enables an analysis that is free from potential bias from inappropriate T2- or T2*-weighting or external technical factors, thereby enabling an objective and automated approach. By using parametric mapping the invention provides a quantitative or semi-quantitative approach towards kidney injury diagnosis. Until the present time the morphology of the kidney and/or the relative signal intensities (gray scale) of T2- or T2*-weighted images may have been assessed and subsequently a subjective decision taken on the presence or absence of a kidney injury. Such a subjective assessment is clearly biased by the potential artefacts described above. The parametric approach as described herein enables a systematic quantification of kidney parameters that are sensitive to blood oxygenation and allows objective diagnosis based on quantitative read-outs of the various parametric maps obtained. This approach therefore allows quantitative comparison between patients with each other or to healthy controls, thereby enabling a numerical read-out of kidney injury intensity. The parametric mapping thereby removes potential bias. Previous approaches disclosed in the art have provided no indication of comparative or quantitative approaches towards kidney injury diagnosis.

A preferred embodiment of the invention therefore relates to a method as described herein, whereby the AKI is an ischemic and/or reperfusion (I/R) renal injury. The use of T2- and/or T2*-weighted MRI to determine I/R AKI represents a novel and particularly advantageous embodiment of the invention. Until presently no effective method has been described in the art that enables quantitative and semi-automated analysis of I/R AKI immediately after injury.

One embodiment of the invention relates to a method as described herein, whereby the AKI is a consequence of kidney hypoperfusion and/or interruption of blood flow.

MRI is not being used in routine clinical diagnosis of kidney diseases and hence is not a technique that might be thought of as a potential replacement for conventional diagnostic techniques for AKI. There is also no obvious link previously disclosed in the art between the MR parameters T2/T2* and the clinical molecular biomarkers, such as NGAL expression. Existing biomarkers focus on impaired kidney function, while the MRI-based method of the invention indirectly measures (patho)physiological status of the kidney. There is therefore no suggestion for a skilled person in the art that the method of the present invention would provide a useful comparison to molecular biomarkers for AKI or in fact a useful replacement and improvement over the methods presently known that rely on detection and/or quantification of molecular biomarkers.

The method of the present invention enables essentially the function of the kidney to be transported into almost real-time images that can be easily quantified in an objective manner immediately after AKI. Biased image brightness (white or black indicates) is converted into objective MR parameters, whose correlations with kidney tissue properties provide information about kidney function and/or status, enabling simple and quick diagnostic procedures.

A preferred embodiment of the present invention relates to a method as described herein, whereby defined regions-of interest (ROI) within the cortex and/or medulla, preferably the renal cortex, outer medulla and/or inner medulla, are imaged and used for quantitative or semi-quantitative determination of T2* and/or T2 values. Through positioning defined regions of interest in relevant physiological domains of the kidney effective diagnostic results can be obtained. The definition of ROI reduces subjectivity in the method, which could be introduced according to the individual user. The invention enables semi-automated approaches to quantifying AKI without the user of the device being required to make a subjective assessment as to the severity of the condition.

In a preferred embodiment the method of the invention is **characterised in that** a segmentation model consisting of three to nine ROIs at defined relative positions within a reference frame are used, whereby the positions of said ROIs are preferably defined as three ROIs in the renal cortex (COR), three ROIs in the outer medulla (OM) and three ROIs in the inner medulla (IM). This particular configuration has proven advantageous due to the relative positioning of the ROIs and the reliable reduced "contamination" from other regions of the kidney.

The standardized ROIs represent an important aspect of the invention and provide an important advancement in terms of quantification of results leading to a more reliable diagnosis. Systematic approaches are enabled based on selecting defined regions of the cortex and medulla for analysis.

In a preferred embodiment the method of the invention is **characterised in that** a drop in T2* and/or T2 values in comparison to a control measurement indicates the presence of ischemic AKI. Interestingly, in the medulla, especially in the outer medulla, the T2 values are reduced over an extended period of time after ischemia, whereby even after reperfusion the oxygen levels are not increased to the same levels as before ischemia was induced. This region is especially relevant for the function of the kidney and is responsible for resorption of fluids after passive filtration (fluid extraction) of the blood in the glomeruli located in the renal cortex. This region is particularly important for AKI diagnosis, as the feature of maintained reduced oxygenation in the medulla as described by reduced T2 or T2*-weighted values provides a persisting and determining indication of an AKI having had occurred. This region of the kidney is also the region most quickly damaged in the context of AKI and therefore provides an important diagnostic readout.

Measurements on healthy subjects may be used as controls in the diagnostic method. Due to variation in natural populations of kidney oxygenation the most effective manner of diagnosis is to use specific control groups based on for example age, size and/or pre-known medical conditions, and to generate average T2- and T2*-values for each of these groups. Statistically significant deviations from the norm-values will then indicate the presence of AKI.

The experimental examples of the invention demonstrate reliable changes in T2* and/or T2 values in direct correlation with the timing and severity of the changes in oxygenation induced via various methods. The method as described herein therefore enables estimation of the staging of AKI, in addition to the detection of its presence. Due to the correlations between kidney oxygenation and AKI severity and/or timing, significant insights into diagnostic criteria for AKI have been gathered using the method described herein.

The invention also relates to a non-invasive method for the early diagnosis of acute kidney injury (AKI) comprising T2- and/or T2*-weighted MRI. Early diagnosis relates preferably to application of the method at time points 48 hours or earlier after AKI. In a preferred embodiment the method is capable of detecting AKI when carried out at time points 6 hours or earlier after injury.

Current methods based on molecular or immunological approaches for AKI diagnosis are significantly slower than those methods described herein. The development and processing of an immune assay (for example an ELISA) or for carrying out a diagnostic PCR is more time intensive and less reliable than the immediate diagnostic possibilities offered by the method of the present invention. The method as described herein allows immediate analysis of a patient or subject who may be at risk or who is suspected of AKI without any delay in processing test results. Due to the automated nature of the analysis, the results may be achieved extremely early in diagnosis of the disease, thereby enabling treatment before serious damage to the kidneys or the rest of the subject's body has occurred due to the kidney malfunction.

The invention therefore relates to a method as described herein, whereby the method is carried out immediately after AKI, for example at or between time points of 30 seconds, 1 minute, 5, 15, 60, 100, 200 or 250 minutes after AKI. The method of the present invention is also able to detect AKI after significantly longer times, which is also shown in the experimental examples. A particular advantage is however the ability to obtain an early diagnosis if required. Methods of the prior art, especially those based on biomarker expression, are typically only possible at the very earliest at 6 hours after injury. Additionally, the carrying out of molecular tests is significantly longer than carrying out a standard objective MRI method according to the present invention.

The invention also relates to an MRI based method as described herein, whereby one or more additional MRI analyses selected from the group consisting of T1-weighted, proton density, magnetization transfer ratio, measures of water diffusion, water content, blood perfusion, sodium and chloride concentration are carried out for the characterization of pathophysiological changes in the kidney. Additional analyses may provide a more comprehensive analysis of the physical state of the kidney and together with the T2* and/or T2 values provide a more comprehensive diagnosis.

The additional MRI analyses can provide complementary information about the pathophysiology of the kidney, which might be necessary to establish an unambiguous relationship between MRI and established biomarkers or pathophysiological status of the kidney.

Ways of combining such additional MRI analyses would include:
1) multi-parametric criteria (such as RIFLE);
2) mathematical combination of MR readouts into a new disease index; and/or
3) automated classification of disease status with multi-parametric MR data as input (e.g. neural network classification or clustering algorithms).

The method of the invention also encompasses additional analysis of one or more molecular biomarkers, preferably KIM-1 and/or NGAL, is carried out, whereby a correlation between the T2- and/or T2*-weighted MRI analysis and a change in the amount of molecular biomarker indicates AKI. The invention therefore also relates to the use of calibrated quantitative MRI for the early diagnosis of AKI, wherein non-specific MR data is combined with kidney injury specific molecular biomarkers.

In a preferred approach the invention employs additional data obtained from preclinical research with animal models of AKI in order to calibrate quantitative MRI methods, so that these can be used as clinical diagnostic biomarkers. For the calibration, established animal models of AKI, such as ischemia-reperfusion (I/R), will preferably be used to:
- induce different degrees of AKI under controlled conditions
- monitor MRI parameters before and during AKI
- assess AKI severity using creatinine and novel molecular biomarkers, such as NGAL, KIM-1, or Cystatin C in serum
- assess AKI severity by measuring the amount of novel molecular biomarkers, such as NGAL and KIM-1 in kidney biopsies (only feasible in preclinical animal experiments)
- monitor arterial blood pressure (ABP) which is equivalent to renal perfusion pressure (RPP), total blood flow to the kidney (renal blood flow, RBF), local flux of erythrocytes (LFC) and local oxygen tension (POC), local flux of erythrocytes (LFM) and local oxygen tension (POM) (except for ABP only feasible in preclinical animal experiments) and/or
- monitor concentration of deoxygenated and oxygenated hemoglobin using near infrared oximetry (only feasible in preclinical animal experiments).

Optionally, clinical data (MRI, creatinine, diuresis, novel molecular biomarkers) from patients with AKI and healthy volunteers is preferably be used in addition to the preclinical data.

The preferred combination of the established marker for AKI (especially serum creatinine), novel molecular biomarkers applied directly to kidney tissue, with MR imaging will allow for the first time to establish a correlation between changes in MRI parameters and the degree of AKI. The invention therefore relates to the application of parametric mapping of the MR biomarkers such as T2, T2*, T1, proton density, magnetization transfer ratio, but also MRI derived measures of water diffusion, water content, blood perfusion, sodium and chloride concentration for the characterization of pathophysiological changes in the kidney.

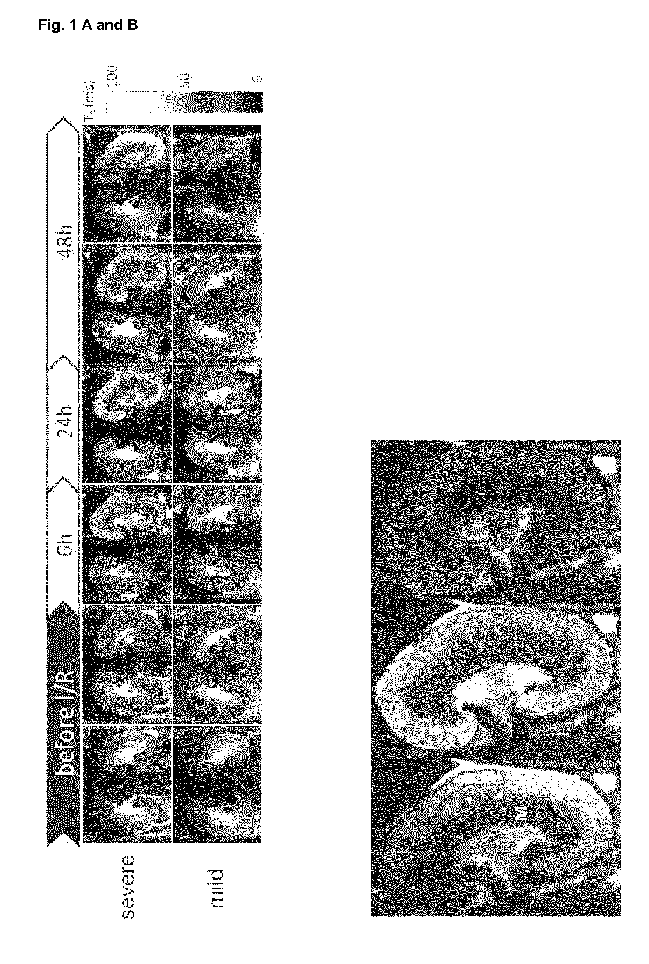

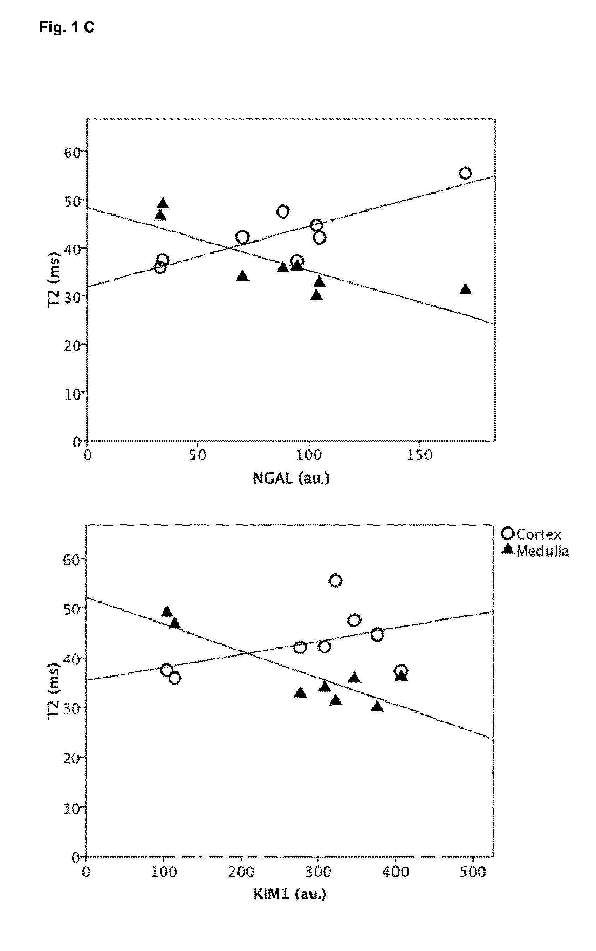

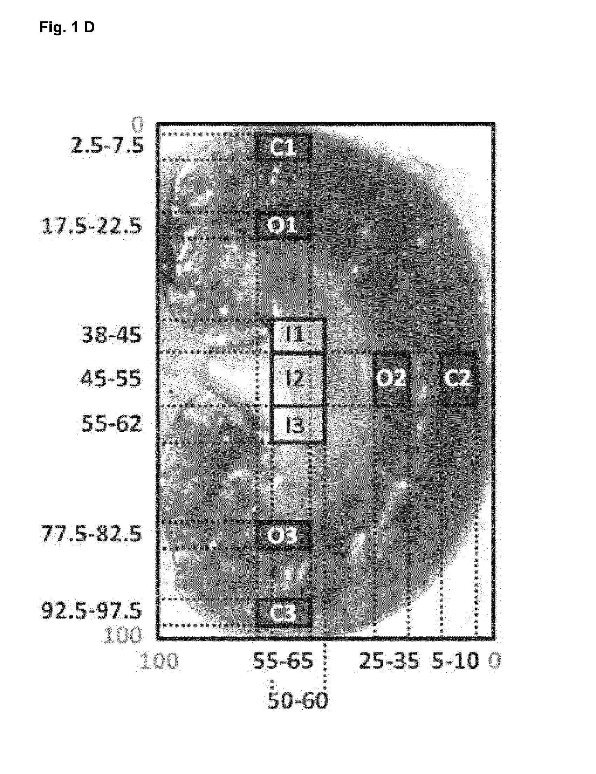

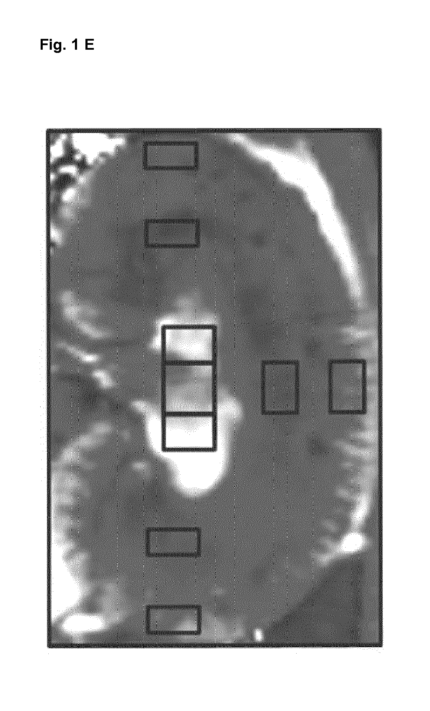

The inventor has shown in studies using an I/R mouse model of AKI (unpublished to date) that the MR biomarker T2 correlates well with NGAL and KIM-1 levels in the tissue of the affected kidney (Figure 1).

Changes in T2 were shown as early as 6 hours after ischemia, but further studies carried out by us even suggest that significant changes in T2 and other MR parameters may be detectable within minutes of ischemia or reperfusion. The data acquisition for each of the MR biomarkers requires merely between 30 seconds and 10 minutes, so that a combination of different MR biomarkers could be used for the proposed MR-based diagnostic method.

In contrast to the existing diagnostic techniques, quantitative MRI of the kidneys enables provision of highly specific information very rapidly, non-invasively and with high sensitivity.

This invention combines for the first time, essentially non-specific MR data with kidney injury specific molecular biomarkers, as well as the established and routinely used kidney injury markers creatinine and diuresis. The proposed diagnostic method or use of calibrated quantitative MRI for the early diagnosis of AKI has the potential to unify the advantages of MR imaging with the specificity of and available clinical knowledge about existing molecular markers for kidney injury.

### DETAILED DESCRIPTION OF THE INVENTION

Parametric MRI can provide maps of the MR relaxation parameters T2* and T2, which are known to be blood oxygenation level-dependent (BOLD) [13]. The BOLD effect relies on the paramagnetic property of deoxygenated hemoglobin. A signal attenuation in T2*-weighted MR images occurs if the volume fraction of deoxygenated hemoglobin increases. T2* or its reciprocal value (R2* = 1/T2*) can be used as a surrogate of renal (blood) oxygenation. Parametric maps of absolute T2* and T2 values can be calculated using a series of MR images acquired at different echo times. For each image pixel a mono-exponential function can be fitted to the signal intensities at different echo times, yielding time constants (T2*/T2) that quantitatively describe the MR properties of the local tissue. The T2*- and T2-weighted images series are acquired with multi-echo gradient-echo or multi-echo spin-echo MR pulse sequences respectively.

Since it is important to diagnose kidney injuries as fast as possible, there is a requirement that diagnostic methods can be carried out accurately and quickly, in order to provide medical practitioners with reliable information obtained in as quickly a manner as possible. Therefore it is a great advantage that the method of the invention can be adapted for use in an automated system or semi-automated system. This embodiment not only realises saving of time, but also of material, work steps and costs.

Clinically acute kidney injury (AKI) is generally defined as a sudden impairment of kidney function, often as a rapid reduction in the glomerular filtration rate (GFR) [references a1, a2]. The causes for AKI are either pre-renal (insufficient blood flow), renal (direct kidney damage), or post-renal (impairment of urine drainage) [a1, a8]. Hence, the possible causes for AKI are numerous and include impaired cardiac function (such as in acute myocardial infarction), obstructions of ureter or bladder (e.g. nephrolithiasis (stones), hypertrophy of the prostate), and hypovolemia. The latter describes a significant loss of blood volume, caused for instance by accidents or excessive loss of gastrointestinal fluids (e.g. diarrhea, vomiting) or interstitial fluid by burn injuries.

An "acute kidney injury" or "acute renal tubular cell injury" includes acute ischemic renal injury (IRI), acute nephrotoxic renal injury (NRI) in addition to renal injury (RI) of other origins. These phrases may be interchangeably used. Although the examples provided herein deal primarily with Hypoxia, Hyperoxia or Ischemia/Reperfusion experiments, the findings are also relevant for nephrotoxic renal injury and renal injury of other origins due to the similarities in kidney response in all types of injury. IRI includes but is not limited to ischemic injury, chronic ischemic injury, acute renal failure and acute tubular necrosis. Further renal injuries of other origins include but are not limited to acute glomerulonephritis and acute tubulo-interstitial nephropathy. NRI toxicity includes but is not limited to, sepsis (infection), shock, trauma, kidney stones, kidney infection, drug toxicity, poisons or toxins, or after injection with a radiocontrast dye. Kidney injuries of the invention relate to all disease mentioned in N17 - N19 of the WHO classification.

As used herein the expression "renal tubular cell injury" especially means a renal or kidney failure or dysfunction, either sudden (acute) or slowly declining over time (chronic), that can be triggered by a number of disease or disorder processes. Both acute and chronic forms of renal tubular cell injury can result in a life-threatening metabolic derangement.

Acute renal or kidney injury may in some cases lead to chronic kidney disorders. The phrases "chronic renal tubular cell injury", "progressive renal disease", "chronic renal disease (CRD)", "chronic kidney disease (CKD)" as used interchangeably herein, especially include any kidney condition or dysfunction that occurs over a period of time, as opposed to a sudden event, to cause a gradual decrease of renal tubular cell function or worsening of renal tubular cell injury. One endpoint on the continuum of chronic renal disease is "chronic renal failure (CRF)". For example, chronic kidney disease or chronic renal injury as used interchangeably herein, includes, but is not limited to, conditions or dysfunctions caused by chronic infections, chronic inflammation, glomerulonephritides, vascular diseases, interstitial nephritis, drugs, toxins, trauma, renal stones, long standing hypertension, diabetes, congestive heart failure, nephropathy from sickle cell anemia and other blood dyscrasias, nephropathy related to hepatitis, HIV, parvovirus and BK virus (a human polyomavirus), cystic kidney diseases, congenital malformations, obstruction, malignancy, kidney disease of indeterminate causes, lupus nephritis, membranous glomerulonephritis, membranoproliferative glomerulonephritis, focal glomerular sclerosis, minimal change disease, cryoglobulinemia, Anti-Neutrophil Cytoplasmic Antibody (ANCA)-positive vasculitis, ANCA-negative vasculitis, amyloidosis, multiple myeloma, light chain deposition disease, complications of kidney transplant, chronic rejection of a kidney transplant, chronic allograft nephropathy, and the chronic effects of immunosuppressives. Preferably, chronic renal disease or chronic renal injury refers to chronic renal failure or chronic glomerulonephritis.

One example of acute kidney injury relates to post-operative kidney failure, a disorder that is exhibited in patients after surgical operation. Some operations are more inclined to be followed by acute kidney injury, such as in children who have undergone Cardiopulmonary bypass surgery (Krawczeski,et al, 2001, Journal of Pediatircs). The diagnosis of kidney injury in such cases is of utmost important, so that appropriate measures can be taken regarding therapeutic treatment.

The expression level of one or more molecular biomarkers may be used to confirm or support the diagnosis by MRI. The terms determination or measuring of "expression level" relates to the measurement or analysis of any indicator of biomarker expression, regarding preferably the measurement of biomarker presence. Expression level of a biomarker may include gene expression or activity, such as direct measurements of transcription, such as RNA production or RNA accumulation either intra- or extra-cellular, or indirect measurements of transcription, for example analysis of chromatin environment surrounding the gene coding for the biomarker, thereby indicating whether transcription of the biomarker gene is ongoing and to what extent. Chromatin modifications and their relationship to gene transcription are known in the art. Protein expression or accumulation is also encompassed within "expression level", either of intra- or extra-cellular protein. According to the central dogma of genetic information, as is understood by those skilled in the art, the flow of information from gene (DNA) to RNA to protein is linked, so that the biomarker expression level may relate to measurement of the level of any molecule related to the expression or production to any of the herein described biomarkers.

As used herein, the terms "subject" and "patient" are used interchangeably irrespective of whether the subject has or is currently undergoing any form of treatment. As used herein, the terms "subject" and "subjects" especially refer to a mammal including, a non-primate (for example, a cow, pig, camel, llama, horse, goat, rabbit, sheep, hamsters, guinea pig, feline, canine, rat, and murine), a non-human primate (for example, a monkey, such as a cynomolgous monkey, chimpanzee, etc) and a human. Preferably, the subject is a human.

### FIGURES

The figures provided herein represent examples of particular embodiments of the invention and are not intended to limit the scope of the invention. The figures are to be considered as providing a further description of possible and potentially preferred embodiments that enhance the technical support of one or more non-limiting embodiments.
**Figure1A****:** Changes in T2 alter 1/R demonstrated in the form of grayscale-coded T2-maps for severe AKI (top row) and mild AKI (bottom row) of the left kidney. The grayscale-coded maps of the kidneys were superimposed to the T2-weighted images. The corresponding T2-weighted anatomical images (grey) am shown for the fast and last time point, i.e. before 1/R and 48h after 11K. The grayscale used in the T2-maps ranged from 0 to 100ms (right).
**Figure 1B****:** T2-weighted image of a clipped kidney showing the ROI placement (left), the corresponding T2-map (centre) and T2-map (right).
**Figure 1C****:** T2 of the cortex (circles) and medulla (black triangles) of the clipped kidney (alter 48h) vs. NGAL (left) and vs. KIM I (right). Black lines represent the fitted linear regression curve.
**Figure 1D****-E:** Standardized segmentation model of the rat kidney that is used to place regions-of-interest (ROI) in the cortex (C1,C2,C3), the outer medulla (O1,O2,O3) and the inner medulla (I1,I2,I3). D) Segmentation model overlaid onto a photograph of a freshly excised coronal view of a rat kidney. During analysis the rectangular reference frame is manually positioned around the kidney in the coronal view. The ROIs are placed automatically at pre-defined relative positions within this reference frame (number ranges signify percentages of the reference frame dimensions). E) Example of a color-coded T2* parameter map, showing a rat kidney in-vivo together with an overlay of the segmentation model.

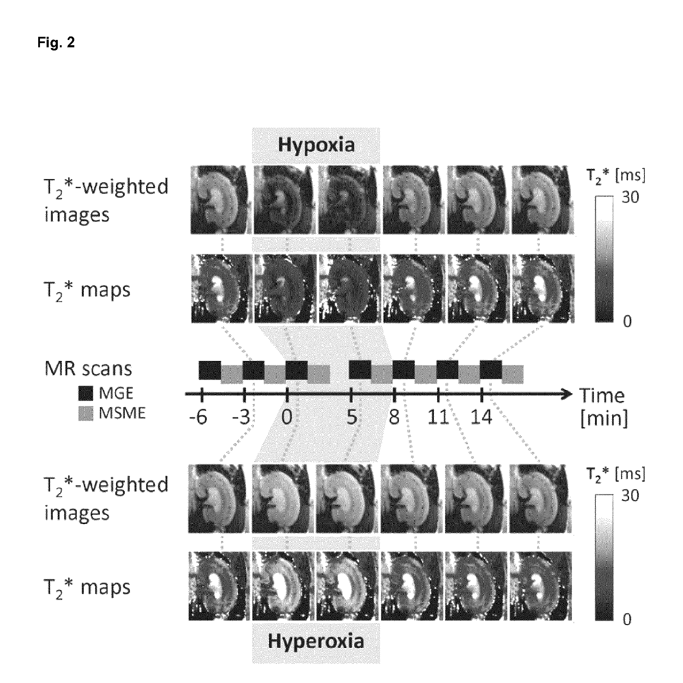

**Figure 2****:** Hypoxia and hyperoxia experiments: T2*-weighted MR images (gray scale) and T2* parameter maps (color-coded) derived from MGE acquisitions. Following two baseline measurements of T2*, hypoxia/hyperoxia was induced by changing the inhaled gas mixture from air to 10%/90% O2/N2 or 100% 02. T2*/T2 measurements were repeated directly after and exactly 5 minutes after onset of hypoxia/hyperoxia (to ensure equal timing of the MR scans). Subsequently inhaled gas was changed back to air and T2*/T2monitored continuously for another 9 minutes.

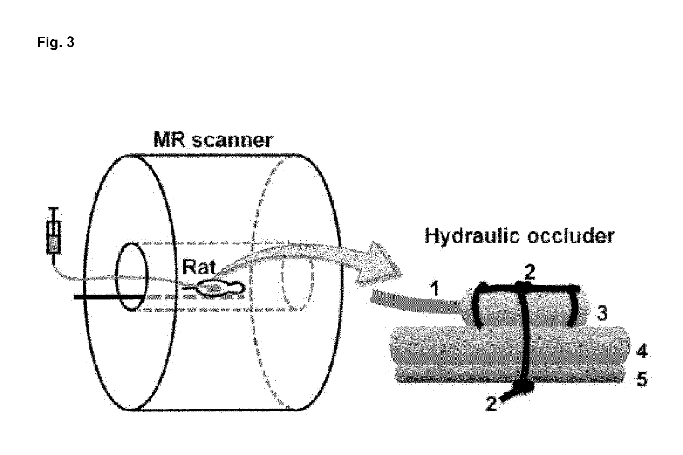

**Figure 3****:** Schematic view of the hydraulic occluder (right) used for induction of renal ischemia during MRI. 1 = indistensible extension tube, 2 = sutures, 3 = distensible silicone tube, 4 = renal vein, 5 = renal artery. A water-filled syringe, connected to the indistensible extension tube, is used to create a hydraulic pressure, which leads to an inflation of the distensible tube. This causes a compression of the renal artery and vein and restricts the blood flow.

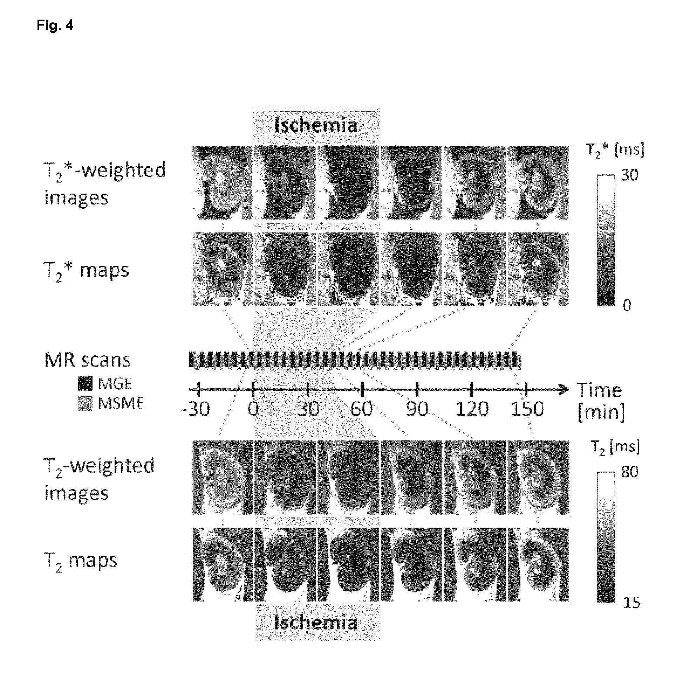

**Figure 4****:** Ischemia reperfusion experiments: T2*-weighted (MGE acquisition) and T2-weighted MR images (MSME acquisition) shown in gray scale together with grayscale-coded T2* and T2 parameter maps. Alternating T2* and T2 measurements (MGE and MSME scans) were repeated continuously throughout the entire experiment (55 time points). Images and maps are shown for six time points: last baseline, onset of ischemia and end of ischemia after 45 minutes, onset of reperfusion, and 12 minutes and 100 minutes after onset of reperfusion.

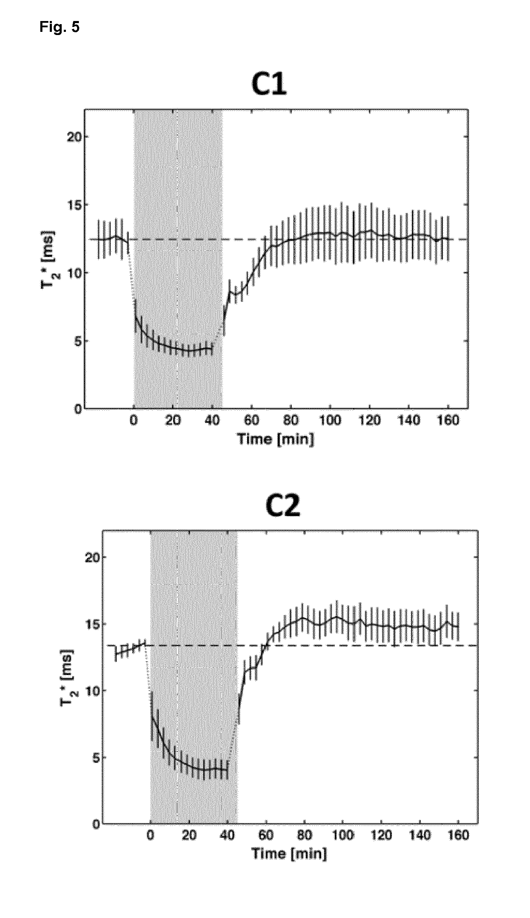

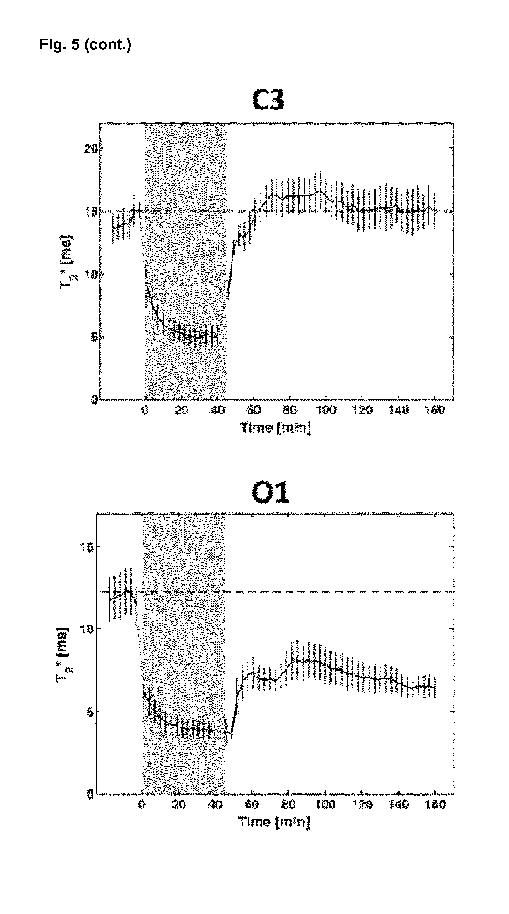

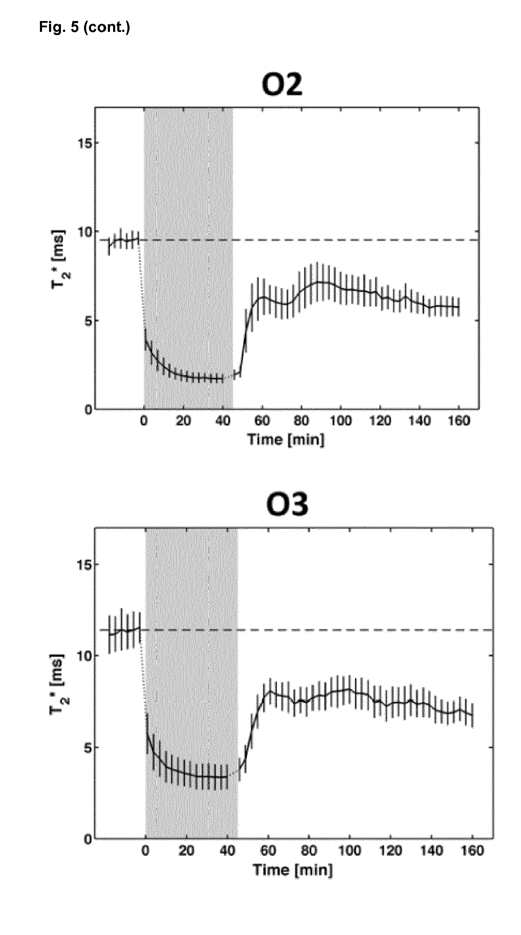

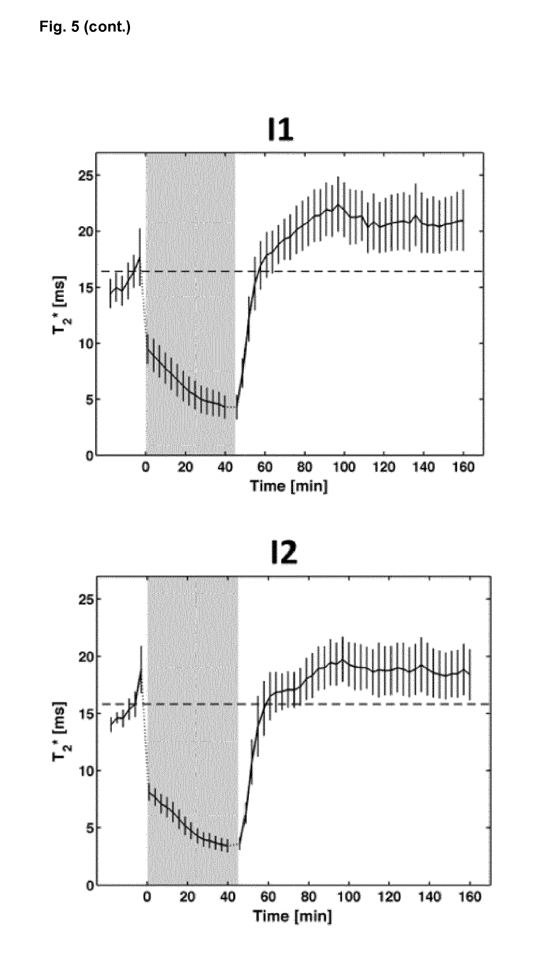

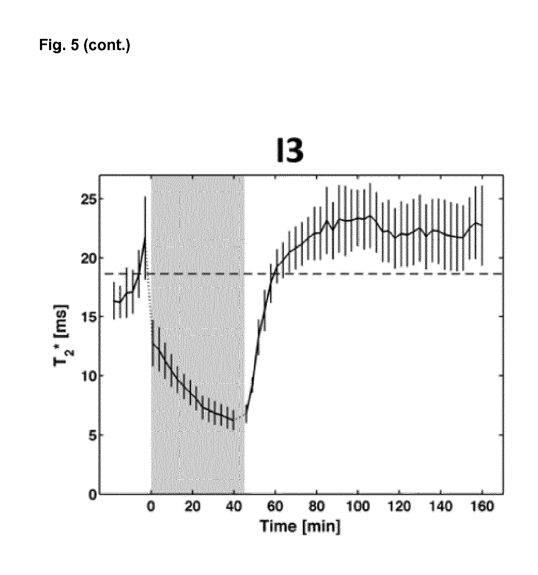

**Figure 5****:** Ischemia reperfusion results derived from the standardized segmentation model of the kidney: Plots of T2* (mean 6SEM averaged over six animals) versus time for the ROIs in the cortex (C1, C2, C3), in the outer medulla (O1, O2, O3), and in the inner medulla (I1, I2, I3). Ischemia (shaded in gray) led to an immediate and significant T2* decrease in all kidney ROIs (p≤0.01 in C1, p≤0.001 in all other ROIs). Clear differences between cortex, outer medulla and inner medulla were observed. At the end of the reperfusion period T2* was close to baseline (dashed line) in the cortex (p>0.2), below baseline in the outer medulla (p≤0.05 (C1), p≤0.001 (C2), p≤0.01 (C3)), and above baseline in the inner medulla (non-significant). The three ROIs within each kidney regions showed very similar but not identical trends.

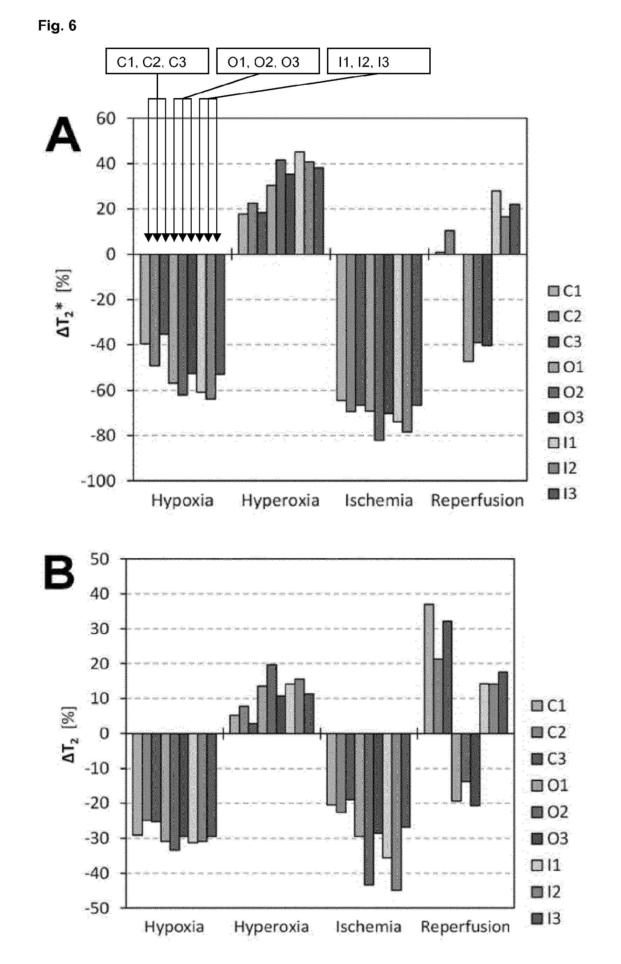

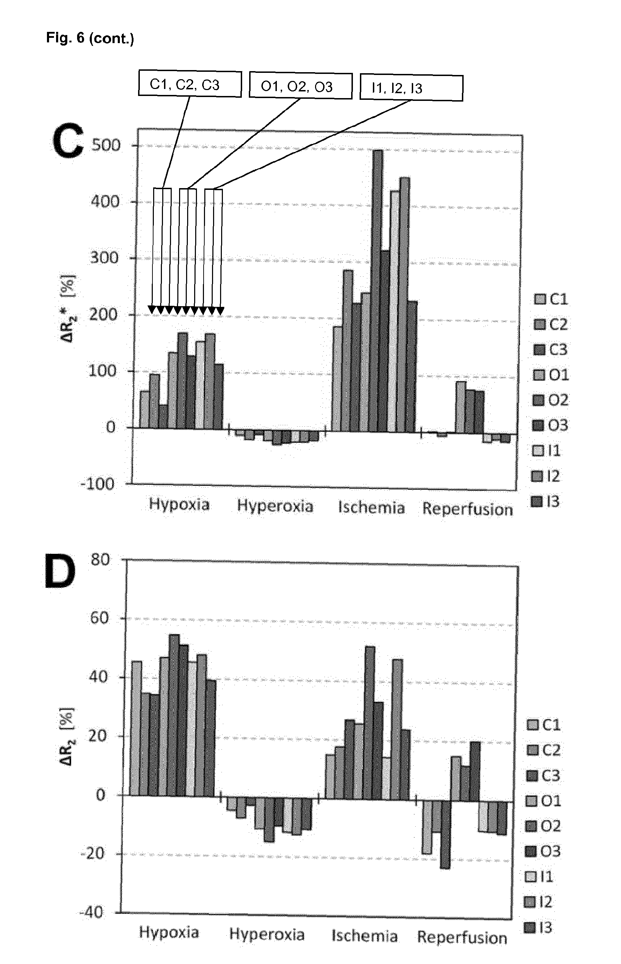

**Figure 6****:** Synopsis of the hypoxia/hyperoxia and ischemia/reperfusion results. Mean changes DT2* (a), DT2 (b), DR2* (c) and DR2 (d) in percent of baseline for all regions-of-interest in the cortex (C1, C2, C3; blue), in the outer medulla (O1, O2, O3; red) and inner medulla (I1, I2, I2; green). Shown are the values for end-hypoxia, end-hyperoxia, end-ischemia and end-reperfusion.

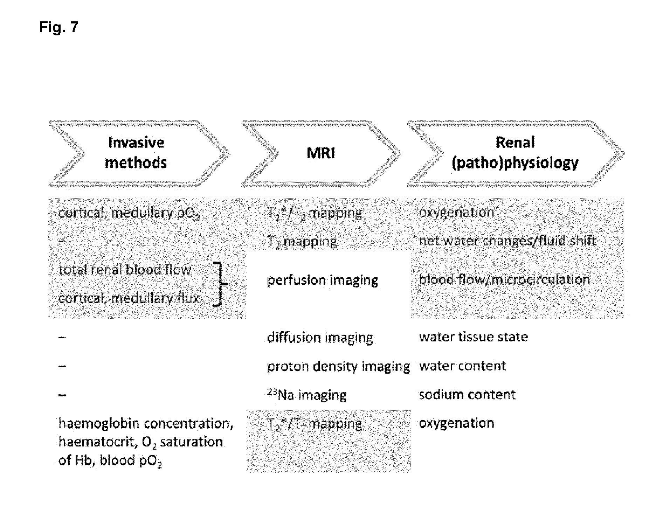

**Figure 7****:** Comparing Invasive Methods with MRI

### EXAMPLES

The examples provided herein represent practical support for particular embodiments of the invention and are not intended to limit the scope of the invention. The examples are to be considered as providing a further description of possible and potentially preferred embodiments that demonstrate the relevant technical working of one or more non-limiting embodiments.

### Example 1: Assessment of AKI severity in an ischemia/reperfusion mouse model using T2 mapping: Comparison with Neutrophil Gelatinase Associated Lipocalin (NGAL) and Kidney Injury Molecule-1 (KIMI).

It is known that renal medullary hypoperfusion and hypoxia play a pivotal rote in acute kidney injury (AKI). A widely accepted hypothesis is that AKI of various origins share one common link in the pathophysiological chain of events that ultimately leads to AKI: Imbalance between renal medullary oxygen delivery and oxygen demand. MR1 is non-invasive and supports longitudinal studies. Hence it is an ideal candidate to examine renal tissue oxygenation and oedema. This example examines the correlation between MRI and NGAL/KIMI in mouse AKI including I/R injuries of different severity (mild to severe) to elucidate the link between changes in T2 and T2* and kidney physiology.

We imaged 8 male C57BL6 mice (aged 12 weeks) *in vivo* under isoflurane anesthesia (1.8-2.2% in 100% air). Four animals underwent mild ischemia/reperfusion (17.5 min). In another four animals severe ischemia/reperfusion (30 min) was induced. For all animals the same MR protocol was used prior to onset of ischemia and 6h, 24h and 48h after ischemia/reperfusion. All procedures were performed under normothermic conditions.

*MR imaging:* Coronal T2-W images (RARE (factor 12), TR/TE = 3600ms / I6ms, FOV/matrix/res = (26.7x21)mm² / 256x202 / 0.104x0.104mm. T2 mapping: MSME, TRJTE/FA = 1000ms / 10-70ms (7 values), FOV/ matrix /res = 26.7x42.0mm / 213x338 / 0.124x0.124mm) were acquired on a 9.4T Broker Biospec (Ettlingen Germany) using a four-element mouse cardiac optimized surface toll array and respiratory triggering. For T2* mapping a multi-echo gradient echo, (TRJTE/FA = 100ms / 2.85-28.5ms (10 values) / 22°, FOV/ matrix /res = 26.7x21mm / 215x169 / 0.1 2x0.12mm) was applied. Two slices of 0.65 mm thickness were acquired in all cases. The NGAL and KIM1 mRNA expression was assessed by quantitative real-time polymerase chain reaction (qPCR), based on post mortem biopsies.

T2/T2* maps were derived from multi-echo spin-echo and multi-echo gradient echo data using MATLAB. Statistical analysis was conducted using SPSS20. Correlations were calculated based on mean values obtained from regions of interest in the renal cortex and medulla (Fig.1C).

T2* and T2-weighted images showed strong changes in the cortex-medulla contrast (Fig. 1A and Fig. 1B). A closer examination revealed a decrease in T2*/T2 for the medulla. T2 was found to be approximately 46.5±1.0 ms (mean±SEM) before I/R, 36.9±0.9 ms every 6h of UR and 35.8±1.2 ms alter 48 h of I/R. In comparison an increase in T2*/T2 was observed for the cortex (Fig. 1A and Fig.1B). Here, a T2 of approximately 34.0±0.5 ms was observed prior to UR. 6 h after I/R T2 was 42.9±0.8 ms while a T2 46.8±0.8 ms was obtained alter 48 h of I/R. Even for the mild I-R model significant T2*/T2 changes were observed, which were not as pronounced as those found for the for severe I-R model. The analysis of the T2-values derived from the medulla and cortex of clipped kidneys showed a strong correlation with NGAL (r 0.82, p < 0.05 and r = 0.85, p < 0.01 respectively) as demonstrated in Figure 1C. A similarly strong correlation was observed between KIMI and T2 deduced from the medulla (r = -0.87, p < 0.01) bot not between KIM1 and T2 deduced from the cortex (r = 0.46) of clipped kidneys (Fig.1C). These results suggest that *in-vivo* parametric MRI (i.e. T2) correlates with the expression of NGAL and KIMI. This indicates that T2-mapping is useful as a non-invasive marker for the characterization of pathophysiological changes in mouse models of AKI.

In order to further refine the parameters for early detection of AKI changes in renal T2*, T2 were explored during variations of inspiratory gas composition (hypoxia, hyperoxia) and during renal ischemia/reperfusion.

### Example 2: Hypoxia and Hyperoxia Experiments.

The variations in inspiratory gas composition demonstrated a high sensitivity of T2* and T2 to renal blood oxygenation changes. Parametric mapping revealed a decrease in T2* and T2 for hypoxia and an increase for hyperoxia in all kidney layers (Fig. 2). For quantitative examination of T2* and T2 changes, mean values were determined for nine regions-of-interest (ROI) according to the proposed standardized renal segmentation model. Under hypoxic conditions T2* decreased by 35-49% (COR), 53- 62% (OM), and 53-64% (IM), while T2 decreased by 25-29% (COR), 29-33% (OM), and 29-31% (IM). The observed increase during hyperoxia was 18-23% (COR), 31-42% (OM), 38-45% (IM) for T2* and 3-8% (COR), 11-20% (OM), 11-16% (IM) for T2.

### Example 3: Ischemia/Reperfusion Experiments

T2* and T2 were monitored before and during ischemia/ reperfusion with a temporal resolution of apr. 3 minutes. Figure 4 shows T2*-weighted and T2-weighted MR images for six time points, together with T2* and T2 parameter maps. The six time points represent last baseline, onset of ischemia and end of ischemia after 45 minutes, onset of reperfusion, and 12 minutes and 100 minutes after onset of reperfusion. These parametric maps demonstrated immediate changes in T2*/T2 after onset and end of ischemia.

Quantitative examination of T2* and T2 changes used the standardized renal segmentation model. Plots of T2* (mean 6SEM averaged over six animals) versus time for these ROIs are shown in Figure 5. The T2* time courses revealed an immediate drop in T2* after onset of ischemia followed by a slow and steady decrease in T2*. During ischemia T2* was significantly reduced in all ROIs (p≤0.01 in C1, p≤0.001 in all other ROIs), which clearly distinguished this condition from baseline. The time courses of T2 were very similar to those of T2*. During reperfusion, T2* and T2 did return to baseline in the cortex, but not in the medulla. While no major differences among the cortical, outer medullary, and inner medullary T2* changes were found during ischemia, there was a clear regional dynamics during reperfusion. After onset of reperfusion T2* returned close to baseline for cortical segments (p = 0.97 (C1), p = 0.22 (C2), p = 0.65 (C3)). For the outer medulla T2* remained below baseline throughout reperfusion and was at 40-47% below baseline after 100 minutes (p≤0.05 (C1), p≤0.001 (C2), p≤0.01 (C3)). T2 was 19-21% below baseline at 100 minutes reperfusion. In the inner medulla T2* and T2 exceeded baseline by 17-28% and 14-18% respectively (non-significant) at the end of reperfusion. These differences among the layers were pronounced during early reperfusion and remained stable throughout reperfusion. The assessment of the heterogeneity among segments within one kidney layer revealed an almost equal time course in T2* for the cortical, outer medullary and inner medullary segments, respectively.

### Example 4: Comparison of Hypoxia/Hyperoxia and Ischemia/ Reperfusion

Changes in renal T2* and T2 during hypoxia, hyperoxia, ischemia and reperfusion were measured using T2* and T2 difference maps.

Hypoxia/hyperoxia led to a rather uniform T2*/ T2 reduction/rise across the kidney. The T2*/T2 reduction in ischemic kidneys exceeded that during hypoxia. Changes in the course of reperfusion clearly differentiated cortex and medulla. In accordance with the ROI data medullary T2*/T2 remained reduced throughout reperfusion, cortical T2* returned to baseline and cortical T2 even rose markedly above baseline.

For a quantitative synopsis of the ischemia-reperfusion results together with the hypoxia/hyperoxia results the MR parameter values at baseline, end-hypoxia/hyperoxia, end-ischemia and end reperfusion were chosen. The group mean T2* and T2 with standard errors (SEM) are surveyed for all regions-of-interest together with the corresponding relativities R2* and R2. Relative changes in these parameters are summarized in Figure 6. DT2* (DR2*) and DT2 (DR2) derived from the outer/inner medulla exceeded those observed in the cortex (except for DT2 (DR2) during reperfusion). For all regions the T2* (R2*) changes during ischemia were more pronounced than during hypoxia. T2 (R2) effects during ischemia were comparable to hypoxia.

### Further discussion of the experimental examples

The examples of the invention demonstrate that continuous and high temporal resolution parametric MRI is feasible for in-vivo monitoring and characterization of ischemia/reperfusion (I/R) induced AKI in rats. Fast T2*/T2 mapping was combined with a remotely controlled I/R model and a segmentation model based semi-automated quantitative analysis. This approach allowed tracking of T2*/T2 in the kidney during I/R with a high spatial and temporal resolution. Our study provides a technical advance in the field, as only snapshot images acquired before and after induction of injury were previously analyzed in I/R models [16-20].

The proposed segmentation model of the rat kidney served to reduce operator-induced variability compared to manually drawn regions-of-interest used in similar studies [16-19]. Taking into account that the histological borders among the kidney layers are rather undulating, extra care was taken to leave a generous spacing between the ROIs in the cortex and the outer medulla, and between the ROIs in the outer and the inner medulla to avoid partial segment effects. Thus, parametric MRI data of these ROIs can be attributed exactly to the respective morphological layer.

T2* and T2 changes were observed for all kidney regions shortly after induction of renal ischemia and during the initial reperfusion phase, which remained stable throughout the monitored reperfusion period. These findings could help to support the hypothesis that key events for the initiation of injury occur immediately after reperfusion, if a link to observations at later time points (e.g. 6-48 hours) can be established in future studies. In this study the surgical/experimental procedures were adapted for performing in vivo I/R experiments inside of an MR scanner; they do not allow for a recovery of the animal and additional MRI at later time points.

Considering the oxygenation-sensitivity of T2* (R2*) measurements during hypoxia, the immediate decline of T2* at the onset of ischemia, and the accordance with reports on invasively measured tissue pO2 during ischemia [12], it is reasonable to attribute the detected T2* (R2*) changes primarily to variations in intrarenal blood oxygenation. Still, T2* and T2 may only be surrogates for physiological parameters of interest, such as renal blood oxygenation. The influence of numerous other physical and physiological parameters on T2*/T2 variations in vivo, and in particular on the absolute values of these parameters, must be taken into account when comparing parametric MRI data with other quantitative measures of blood (or even tissue) oxygenation. For this reason it is conceptually appealing for future studies to combining invasive techniques and non-invasive MRI in a hybrid setup with the goal to calibrate, monitor and interpret parametric MR and physiological parameters by means of standardized interventions.

Absolute values of baseline renal R2* were found to be lower in the inner medulla versus outer medulla and cortex, which is in agreement with previous reports [16,24,25]. This finding does not correspond with tissue pO2 levels measured by invasive methods [26], which showed the lowest pO2 levels in the inner medulla. One reason behind what seems to be a discrepancy is that the blood oxygenation dependence of R2* (T2*) does not relate to the deoxyhemoglobin/oxyhemoglobin ratio, but to the absolute amount of deoxyhemoglobin per tissue volume. Thus, besides being dependent on blood oxygenation, R2* (T2*) is influenced by the blood vessel volume fraction and the hematocrit. Both, vessel volume fraction and hematocrit are low in the inner medulla [27,28].

In our study complete occlusion of the renal artery and vein were confirmed by MR angiography, rendering potentially confounding changes in global renal blood volume unlikely. Two possibly involved physiological factors are alterations in pH and fluid shifts. A significant drop in pH during renal artery occlusion in rats has been reported earlier [22]. By altering the hemoglobin oxygen saturation curve a pH reduction significantly influences the blood oxygenation effect on T2* [14]. To this end, simultaneous MRI mapping and conventional invasive measurements of oxygenation may allow further insights.

During hypoxia and ischemia MRI revealed significantly higher R2* indicating lower renal oxygenation. The observed increases in R2* during ischemia (180-500% of baseline) are comparable with previous reports [20] considering the difference in species (rat versus mouse model), magnetic field strength and analyses techniques. Parametric MRI monitoring, as established in our study, overcomes the limitations of snapshot data acquisitions at a few time points only and greatly enhances the assessment of spatio-temporal evolution of MR biomarkers - particularly in the initial reperfusion phase.

Our findings regarding R2* during I/R show similarities to invasively measured medullary pO2 time courses previously reported [12] and reflect impaired reoxygenation and hypoperfusion in the outer medulla after reperfusion which is related to the so-called "no reflow" phenomenon [29]. Parametric MRI indicated a strong persistent hypoxia in the outer medulla during reperfusion. The outer medulla is particularly susceptible to an ischemic insult due to high cellular metabolic rate (S3 segment of the proximal tubules, thick ascending limbs of Henle's loop) is high and marked hypoperfusion and congestion persist during reperfusion, i.e. in the extension phase of AKI [30]. The R2* effects of hyperoxia on renal oxygenation were much smaller compared to hypoxia, which might be due to the already high arterial oxygen saturation of hemoglobin during normoxia. The amount of oxygen not bound to hemoglobin but dissolved in blood, which is expected to increase during hyperoxia, contributes only a rather small fraction to the overall oxygen supply.

Interleaved mapping of T2* and T2 may provide complementary information about the (patho)physiological conditions in the kidney as both MR parameters are affected by changes in blood oxygenation and water content to different degrees. Variations in the volume fraction of deoxygenated hemoglobin impact on the MR signal stemming from intra-vascular spaces of large vessels or capillaries as well as regions around these vessels. These intra- and extravascular signal components contribute differently to T2* and T2 [31,32] and T2 is sensitive to water content [33]. Hence mapping of both parameters could allow for a more comprehensive assessment of renal oxygenation changes. The strong T2* and T2, changes observed in this study will inspire further explorations into MR methodology suitable for simultaneous T2*/T2 mapping.

Continuous parametric MR monitoring of T2* and T2 throughout an I/R experiment in rats is feasible and provides a temporal resolution of approximately 80-100 seconds for each read-out resulting in 3 minutes for alternating T2*/T2 measurements. The MRI approach described enables the detailed assessment of in vivo changes in all kidney regions during ischemia and early reperfusion. Observations in the early reperfusion phase promise to offer new insights into the pathogenesis of ischemia induced AKI and might help to identify the timeline of key events responsible for development of hypoperfusion-induced renal damage. The established method of parametric MR monitoring holds the promise to be a useful investigational tool for other models of AKI such as x-ray contrast agent induced AKI, rhabdomyolysis-induced AKI, or sepsis-induced AKI.

### Materials and Methods

Animal experiments were carried out in accordance with the guidelines provided and approved by the Animal Welfare Department of the Berlin State Office of Health and Social Affairs (permit numbers G00121/11, G0059/12). All experiments were carried out under urethane anesthesia (20% in saline, 6 ml/kg body mass, intraperitoneal), and all efforts were made to minimize suffering. Animals were kept under constant thermal conditions: 37uC body temperature was maintained by warming the animal beds using a circulating heated water system (Thermo Haake GmbH, Karlsruhe, Germany). Rectal temperature and respiratory motion/ rate were monitored (SA Instruments, Inc., New York, USA) throughout the imaging experiments.

### MR Imaging:

All imaging experiments were carried out on a 9.4T small animal MR system (Biospec 94/20, Bruker Biospin, Ettlingen, Germany) equipped with a linear polarized birdcage RF resonator for transmission in conjunction with a curved four channel receive RF coil array (Bruker Biospin, Ettlingen, Germany) customized for rats.

MRI protocols for parametric mapping of T2* and T2 were tailored for fast measurements in the rat kidney at a magnetic field strength of 9.4 Tesla. T2-weighted pilot scans and local B0 shimming that uses a voxel enclosing the kidney only were performed first. Subsequently, parametric T2* and T2 mapping using respiratory gated imaging techniques was performed. For T2* mapping a multi gradient echo (MGE) sequence (repetition time = 50 ms, echo times = 10, first echo time = 1.43 ms, echo spacing 2.14 ms, averages = 4) was employed with a total acquisition time of approx. 1 min 20 s (respiratory rate dependent). For T2 mapping a multi spin echo (MSME) sequence (repetition time = 550 ms, echo times = 7, first echo time = 10 ms, echo spacing 10 ms, averages = 1) was used with a total acquisition time of approx. 1 min 40 s. A coronal oblique image slice was acquired with a spatial in plane resolution of (2266445) mm2 (field of view = (38.2650.3) mm2 , matrix size = 1696113 zero-filled to 1696215) and a slice thickness of 1.4-1.5 mm.

Renal blood flow during I/R experiments was monitored by time-of-flight (TOF) MR angiographies of the kidney. TOF angiography consisted of an untriggered spoiled gradient echo sequence (2D FLASH, repetition time = 11 ms, echo time = 3 ms, flip angle = 80 degree, spatial in plane resolution of (2006268) mm2) with 15 slices of 1.0 mm thickness placed perpendicular to the major renal blood vessels, acquired in 24 seconds.

### Image Analysis and Kidney Segmentation Model:

Parametric maps of absolute T2* and T2 values were calculated by pixel-wise mono-exponential fitting to the signal intensities of the T2*- and T2-weighted images acquired at different echo times (in-house developed program; MATLAB, R2010a, MathWorks, Natick, WA, USA). The relaxation times T2* and T2 were chosen over the relaxivities (i.e. R2* = 1/T2* and R2= 1/T2), as they allow for a better visualization of changes in the parameter maps for the different kidney regions and stimuli. In quantitative data representations such as value tables or bar charts the relaxation times are complemented by the corresponding relativities, which were also derived on a pixel-by-pixel basis.

Movements of the kidney during I/R experiments were compensated by image registration (FLIRT, FSL, www.fmrib.ox. ac.uk/fsl) using the first echo images of the multi-echo MGE or MSME acquisitions. Images were registered onto a baseline scan and the resulting spatial transformation matrices applied to the corresponding parameter maps.

Mean T2* (R2*) and T2 (R2) values for several regions-of interest (ROI) within the renal cortex, outer medulla and inner medulla were calculated from the parameter maps.

To reduce operator-induced variability in the ROI placement a standardized segmentation model of the rat kidney was developed. For this purpose special care was taken with regard to the location of the ROIs in the kidney. The aim was to determine ROIs strictly according to morphological kidney features, i.e., the distinct renal layers: cortex, outer medulla, and inner medulla (Figure 1). To this end, the dimensions of the rat kidney layers were measured in a series of freshly harvested kidneys as well as in a series of formalin-fixed kidneys (altogether 16 kidneys). To account for the inter-individual variability of the absolute length (cranial to caudal extremities) and width (lateral to medial border) of the kidneys, a rectangular frame that tightly encloses the kidney in the coronal view was used as a reference. The segmentation model consists of nine ROIs at defined relative positions (percentages of both diameters) within this rectangular reference frame (Figure 1). The size and positions of nine ROIs were defined for three ROIs in the renal cortex (COR), for three ROIs in the outer medulla (OM), and for three ROIs in the inner medulla (IM). All ROIs were placed in safe distance from the borders between these kidney layers to avoid any 'contamination' from the neighboring layers (partial segment effects) and to allow for inter-individual variations in morphology without the need to change the ROI position. The implementation of this model in a semi-automated analysis program developed in ImageJ (NIH, Bethesda, MD, USA, http://imagej.nih.gov/ij) limited user interaction to the placement of the rectangular reference frame around the kidney. The automatically placed ROIs were overlaid onto the parameter maps and their position was visually validated. ROIs that included artifacts were excluded from further analysis.

Due to the need for respiratory triggering the time intervals between MRI scans (apr. 180 seconds) varied slightly during the imaging experiments and between animals. To allow for a comparison between different animals the acquired T2* time courses were linearly interpolated and T2* calculated for time points at exact intervals of 180 seconds. Group mean values and standard errors of the mean (SEM) were then calculated for each time point and T2* (mean 6SEM averaged over six animals) plotted versus time. In all other cases (e.g. bar charts of parameter values at end-baseline, end-ischemia, etc) the original, noninterpolated, data were used.

### Hypoxia and Hyperoxia Experiments:

Renal T2* and T2 mapping at 9.4 Tesla in rats under variable physiological conditions has not been reported so far. Hence, the sensitivity of T2* and T2 to renal blood oxygenation changes was demonstrated by examining the changes induced in these parameters by externally controlled variations of blood oxygenation. Blood oxygenation level changes (hypoxia or hyperoxia) were induced in six male rats (Wistar, aged 3-4 month, 300-350 g body weight) by adapting the inhaled gas composition.

Experimental protocol: A respiratory mask was used, which provided air (normoxia) at a flow rate of 1000 ml/min. Following baseline measurements of T2*/T2, hypoxia was induced by adjusting the gas mixture to 10/90% O2/N2. T2*/T2 measurements were conducted throughout 8 minutes after induction of hypoxia. The final T2*/T2 scans during hypoxia were started exactly 5 minutes after hypoxia onset, to ensure equal hypoxia durations and comparable timing of the MR scans. On completion of these MR scans, inhaled gas mixture was changed back to air and T2*/T2 continuously monitored for another apr. 9 minutes. The same protocol was followed for the hyperoxia experiments by setting the inhaled gas to 100% 02. Figure 2 illustrates the experimental procedure, by showing the timeline and the corresponding T2*-weighted MR images together with calculated T2* parameter maps acquired prior, during and after hypoxia/hyperoxia.

### Ischemia/Reperfusion Experiments:

Six male rats (Lewis, aged 2-3 months, 250-300 g body weight) underwent experimental ischemia-reperfusion inside the MR scanner.

Preparation: The abdominal cavity was opened by a midventral incision and the right kidney was removed. A remote controlled hydraulic occluder was placed around the renal artery and vein of the remaining left kidney to allow induction of renal ischemia. The hydraulic occluder (Figure 3) consists of a distensible silicone tube that is connected to a syringe via an indistensible extension tube. A suture loop around the distensible tube and renal vein and artery leads to a compression of the artery and vein when the distensible tube is inflated hydraulically. For intraarterial administration of a vehicle solutions (to be replaced by drugs in future studies) to the kidney a catheter was placed in the aorta with its tip directly at the renal branch. A second remote controlled hydraulic occluder was placed just below the renal branch around the aorta. With the purpose of monitoring the temperature of the kidney throughout the imaging experiment, a fiber-optic temperature probe (OTP-M, AccuSens, Opsens, Quebec City, Canada) was positioned in close proximity to the kidney. The abdominal cavity was then filled with warmed saline and closed by sutures.

Experimental protocol: Directly following surgery the animal was transferred to the warmed MRI animal bed. For the remainder of the experiment T2* and T2 were continuously monitored by interleaved T2*/T2 mapping with a temporal resolution of appr. 3 minutes. After a baseline of five T2*/T2 measurements the hydraulic occluder around the aorta was temporarily closed and a vehicle (100 ml of saline containing 1% DMSO) administered. Thereafter, ischemia was induced by closing the hydraulic occluder around the renal artery and vein for 45 minutes, followed by a reperfusion phase of appr. 100 minutes. Renal blood flow during the three phases of the I/R experiment was monitored by time-of-flight (TOF) MR angiographies of the kidney, acquired prior and immediately after ischemia induction, as well as after onset of reperfusion. The absence of the flow dependent intra-vascular signal during ischemia served to confirm complete occlusion of the renal artery and vein. Figure 4 illustrates the experimental procedure, by showing the timeline and the corresponding T2*-weighted and T2-weighted MR images for six time points, together with T2* and T2 parameter maps.

### Statistical Analysis:

T2* and T2-derived from the nine kidney ROIs were statistically analyzed using SPSS (version 20, IBM Deutschland GmbH, Germany). Data are expressed as mean6SEM. Normal distribution was confirmed by a one-sample Kolmogorov-Smirnov test. Tests of significance (2-tailed t-test) were performed for the last time points in each experimental phase (baseline, ischemia, reperfusion) following Levene's test for equality of variances. A probability value of ≤0.05 was considered significant.

Additionally, further experimentation shows that the preferred embodiments of the invention provide surprising and unexpected effects, thereby solving the problem of the invention in a non-obvious fashion.

### References

a1. Feldkamp, T., et al., Akutes Nierenversagen. Kompendium Nephrologie, 2011. 3(1): p. 6-20.
a2. Herget-Rosenthal, S., Akutes Nierenversagen - Definition und Einteilung. Nephrologie im Dialog, 2011. 2: p. 4-5.
a3. Chertow, G.M., et al., Acute kidney injury, mortality, length of stay, and costs in hospitalized patients. J Am Soc Nephrol, 2005. 16(11): p. 3365-70.
a4. Lassnigg, A., et al., Impact of minimal increases in serum creatinine on outcome in patients after cardiothoracic surgery: do we have to revise current definitions of acute renal failure? Crit Care Med, 2008. 36(4): p. 1129-37.
a5. Mehta, R.L., et al., A randomized clinical trial of continuous versus intermittent dialysis for acute renal failure. Kidney Int, 2001. 60(3): p. 1154-63.
a6. Joannidis, M., et al., Prevention of acute kidney injury and protection of renal function in the intensive care unit. Expert opinion of the Working Group for Nephrology, ESICM. Intensive Care Med, 2010. 36(3): p. 392-411.
a7. Kielstein, J.T., Akute Nierenschädigung. Nephrologie im Dialog, 2011. 2: p. 2-3.
a8. Feldkamp, T., A. Bienholz, and A. Kribben, Akutes Nierenversagen - Ätiologie, Pathophysiologie und Diagnose. Nephrologie im Dialog, 2011. 2: p. 6-8.
a9. Kierdorf, H.P., Nierenersatztherapie bei akutem Nierenversagen. Nephrologie im Dialog, 2011. 2: p. 9-13.
a10. Bagshaw, S.M., et al., A multi-centre evaluation of the RIFLE criteria for early acute kidney injury in critically ill patients. Nephrol Dial Transplant, 2008. 23(4): p. 1203-10.
a11. Coca, S.G., Long-term outcomes of acute kidney injury. Curr Opin Nephrol Hypertens, 2010. 19(3): p. 266-72.
a12. Lafrance, J.P. and D.R. Miller, Acute kidney injury associates with increased long-term mortality. J Am Soc Nephrol, 2010. 21(2): p. 345-52.
a13. Levey, A.S., et al., A more accurate method to estimate glomerular filtration rate from serum creatinine: a new prediction equation. Modification of Diet in Renal Disease Study Group. Ann Intern Med, 1999. 130(6): p. 461-70.
a14. Joannidis, M., et al., Acute kidney injury in critically ill patients classified by AKIN versus RIFLE using the SAPS 3 database. Intensive Care Med, 2009. 35(10): p. 1692-702.
a15. Coca, S.G., et al., Biomarkers for the diagnosis and risk stratification of acute kidney injury: a systematic review. Kidney Int, 2008. 73(9): p. 1008-16.
a16. Herget-Rosenthal, S., et al., Early detection of acute renal failure by serum cystatin C. Kidney Int, 2004. 66(3): p. 1115-22.
a17. Inoue, T., et al., Noninvasive evaluation of kidney hypoxia and fibrosis using magnetic resonance imaging. J Am Soc Nephrol, 2011. 22(8): p. 1429-34.
a18. Kalantarinia, K., Novel imaging techniques in acute kidney injury. Curr Drug Targets, 2009. 10(12): p. 1184-9.
a19. Prasad, P.V., Evaluation of intra-renal oxygenation by BOLD MRI. Nephron Clin Pract, 2006. 103(2): p. c58-65.
a20. Yang, D., et al., Normal and transplanted rat kidneys: diffusion MR imaging at 7 T. Radiology, 2004. 231 (3): p. 702-9.
1. de Mendonca A, Vincent JL, Suter PM, Moreno R, Dearden NM, et al. (2000) Acute renal failure in the ICU: risk factors and outcome evaluated by the SOFA score. Intensive Care Med 26: 915-921.
2. Chertow GM, Burdick E, Honour M, Bonventre JV, Bates DW (2005) Acute kidney injury, mortality, length of stay, and costs in hospitalized patients. J Am Soc Nephrol 16: 3365-3370.
3. Hoste EA, Schurgers M (2008) Epidemiology of acute kidney injury: how big is the problem? Crit Care Med 36: S146-151.
4. Nash K, Hafeez A, Hou S (2002) Hospital-acquired renal insufficiency. Am J Kidney Dis 39: 930-936.
5. Schrier RW, Wang W, Poole B, Mitra A (2004) Acute renal failure: definitions, diagnosis, pathogenesis, and therapy. J Clin Invest 114: 5-14.
6. Bonventre JV, Weinberg JM (2003) Recent advances in the pathophysiology of ischemic acute renal failure. J Am Soc Nephrol 14: 2199-2210.
7. Eltzschig HK, Eckle T (2011) Ischemia and reperfusion-from mechanism to translation. Nat Med 17: 1391-1401.
8. Thadhani R, Pascual M, Bonventre JV (1996) Acute renal failure. N Engl J Med 334: 1448-1460.
9. Whitehouse T, Stotz M, Taylor V, Stidwill R, Singer M (2006) Tissue oxygen and hemodynamics in renal medulla, cortex, and corticomedullary junction during hemorrhage-reperfusion. Am J Physiol Renal Physiol 291: F647-653.
10. Legrand M, Mik EG, Johannes T, Payen D, Ince C (2008) Renal hypoxia and dysoxia after reperfusion of the ischemic kidney. Mol Med 14: 502-516.
11. Bonventre JV, Yang L (2011) Cellular pathophysiology of ischemic acute kidney injury. J Clin Invest 121: 4210-4221.
12. Hoff U, Lukitsch I, Chaykovska L, Ladwig M, Arnold C, et al. (2011) Inhibition of 20-HETE synthesis and action protects the kidney from ischemia/reperfusion injury. Kidney Int 79: 57-65.
13. Silvennoinen MJ, Clingman CS, Golay X, Kauppinen RA, van Zijl PC (2003) Comparison of the dependence of blood R2 and R2* on oxygen saturation at 1.5 and 4.7 Tesla. Magn Reson Med 49: 47-60.
14. Prasad PV (2006) Evaluation of intra-renal oxygenation by BOLD MRI. Nephron Clin Pract 103: c58-65.
15. Pedersen M, Dissing TH, Morkenborg J, Stodkilde-Jorgensen H, Hansen LH, et al. (2005) Validation of quantitative BOLD MRI measurements in kidney: application to unilateral ureteral obstruction. Kidney Int 67: 2305-2312.
16. Rognant N, Guebre-Egziabher F, Bacchetta J, Janier M, Hiba B, et al. (2011) Evolution of renal oxygen content measured by BOLD MRI downstream a chronic renal artery stenosis. Nephrol Dial Transplant 26: 1205-1210.
17. Haque M, Franklin T, Prasad P (2011) Renal oxygenation changes during water loading as evaluated by BOLD MRI: effect of NOS inhibition. J Magn Reson Imaging 33: 898-901.
18. Kusakabe Y, Matsushita T, Honda S, Okada S, Murase K (2010) Using BOLD imaging to measure renal oxygenation dynamics in rats injected with diuretics. Magn Reson Med Sci 9: 187-194.
19. Ji L, Li LP, Schnitzer T, Du H, Prasad PV (2010) Intra-renal oxygenation in rat kidneys during water loading: effects of cyclooxygenase (COX) inhibition and nitric oxide (NO) donation. J Magn Reson Imaging 32: 383-387.
20. Oostendorp M, de Vries EE, Slenter JM, Peutz-Kootstra CJ, Snoeijs MG, et al. (2011) MRI of renal oxygenation and function after normothermic ischemia- reperfusion injury. NMR Biomed 24: 194-200.
21. Terrier F, Hricak H, Berry I, Yen BT, Grodd W (1987) Edema and the lack of blood perfusion produce opposite effects on the magnetic resonance characteristics of acutely ischemic rat kidneys. Invest Radiol 22: 118-125.
22. Terrier F, Lazeyras F, Posse S, Aue WP, Zimmermann A, et al. (1989) Study of acute renal ischemia in the rat using magnetic resonance imaging and spectroscopy. Magn Reson Med 12: 114-136.
23. Vexler VS, de Crespigny AJ, Wendland MF, Kuuvatsuru R, Muhler A, et al. (1993) MR imaging of blood oxygenation-dependent changes in focal renal ischemia and transplanted liver tumor in rat. J Magn Reson Imaging 3: 483- 490.
24. Ries M, Basseau F, Tyndal B, Jones R, Deminiere C, et al. (2003) Renal diffusion and BOLD MRI in experimental diabetic nephropathy. Blood oxygen level-dependent. J Magn Reson Imaging 17: 104-113.
25. dos Santos EA, Li LP, Ji L, Prasad PV (2007) Early changes with diabetes in renal medullary hemodynamics as evaluated by fiberoptic probes and BOLD magnetic resonance imaging. Invest Radiol 42: 157-162.
26. Schurek HJ (1988) [Kidney medullary hypoxia: a key to understanding acute renal failure?]. Klin Wochenschr 66: 828-835.
27. Moffat DB, Fourman J (1963) The Vascular Pattern of the Rat Kidney. J Anat 97: 543-553.
28. Zimmerhackl B, Dussel R, Steinhausen M (1985) Erythrocyte flow and dynamic hematocrit in the renal papilla of the rat. Am J Physiol 249: F898-902.
29. Devarajan P (2006) Update on mechanisms of ischemic acute kidney injury. J Am Soc Nephrol 17: 1503-1520.
30. Sharfuddin AA, Molitoris BA (2011) Pathophysiology of ischemic acute kidney injury. Nat Rev Nephrol 7: 189-200.
31. Reeder SB, Faranesh AZ, Boxerman JL, McVeigh ER (1998) In vivo measurement of T*2 and field inhomogeneity maps in the human heart at 1.5 T. Magn Reson Med 39: 988-998.
32. Uludag K, Muller-Bierl B, Ugurbil K (2009) An integrative model for neuronal activity-induced signal changes for gradient and spin echo functional imaging. Neuroimage 48: 150-165.
33. Marinelli NL, Haughton VM, Munoz A, Anderson PA (2009) T2 relaxation times of intervertebral disc tissue correlated with water content and proteoglycan content. Spine (Phila Pa 1976) 34: 520-524.

## Claims

1. Non-invasive method for the diagnosis of acute kidney injury (AKI) comprising T2-and/or T2*-weighted magnetic resonance imaging (MRI) of one or more kidneys of a subject.

2. Method according to claim 1, whereby the AKI is an ischemic and/or reperfusion (I/R) renal injury.

3. Method according to any one of the preceding claims, whereby the AKI is a consequence of kidney hypoperfusion and/or interruption of blood flow.

4. Method according to any one of the preceding claims, whereby the MRI analysis comprises parametric mapping of T2- and/or T2*-weighted images.

5. Method according to any one of the preceding claims, whereby defined regions-of interest (ROI) within the cortex and/or medulla are imaged and used for quantitative or semi-quantitative determination of T2* and/or T2 values.

6. Method according to any one of the preceding claims, whereby defined regions-of interest (ROI) within the renal layers (e.g. cortex, outer medulla and inner medulla) are imaged and used for quantitative or semi-quantitative determination of T2* and/or T2 values.

7. Method according to any one of the preceding claims, whereby a segmentation model consisting of three to nine ROIs at defined relative positions within a reference frame are used, whereby the positions of said ROIs are preferably defined as three ROIs in the renal cortex (COR), three ROIs in the outer medulla (OM) and three ROIs in the inner medulla (IM).

8. Method according to any one of the preceding claims, whereby a reduction in T2* and/or T2 values in comparison to a control measurement indicates the presence of ischemic AKI.

9. Method according to any one of the preceding claims, whereby the method is carried out at time points 48 hours or earlier after AKI.

10. Method according to any one of the preceding claims, whereby the method is carried out at time points 6 hours or earlier after AKI.

11. Method according to any one of the preceding claims, whereby the method is carried out immediately after AKI, for example at or between time points of 30 seconds, 1 minute, 5, 15, 60, 100, 200 or 250 minutes after AKI.

12. Method according to any one of the preceding claims, whereby one or more additional MRI analyses selected from the group consisting of T1-weighted, proton density, magnetization transfer ratio, measures of water diffusion, water content, blood perfusion, sodium and chloride concentration are carried out for the characterization of pathophysiological changes in the kidney.

13. Method according to any one of the preceding claims, whereby additional analysis of one or more molecular biomarkers for AKI is carried out, whereby a correlation between the T2- and/or T2*-weighted MRI analysis and a change in the amount of molecular biomarker indicates AKI.

14. Method according to any one of the preceding claims, whereby additional analysis of the molecular biomarker KIM-1 is carried out.

15. Method according to any one of the preceding claims, whereby additional analysis of the molecular biomarker NGAL is carried out.
